# EUROPEAN PATENT SPECIFICATION

(11) **EP 2 938 306 B1**
(45) Date of publication and mention of the grant of the patent: **07.03.2018**
(21) Application number: 13868220.8
(22) Date of filing: 11.12.2013
(51) Int. Cl.: A61F 13/56, B31F 1/00, A61F 13/496, A61F 13/15

(54) **METHOD AND APPARATUS FOR FORMING DISCRETE ADJUSTABLE PANT-LIKE DISPOSABLE UNDERGARMENTS**
VERFAHREN UND VORRICHTUNG ZUR FORMUNG EINER DISKRETEN, EINSTELLBAREN HÖSCHENARTIGEN EINWEG-UNTERWÄSCHE
PROCÉDÉ ET APPAREIL POUR FABRIQUER DES SOUS-VÊTEMENTS JETABLES DE TYPE CULOTTE RÉGLABLES ET SÉPARÉS

(30) Priority: 27.12.2012 US 201213727745
(43) Date of publication of application: 04.11.2015
(73) Proprietor: Kimberly-Clark Worldwide, Inc., Neenah, Wisconsin 54956 (US)
(72) Inventor: VOLP, Aaron, Mark, Larsen, Wisconsin 54947 (US); MLINAR, Joseph, A., Appleton, Wisconsin 54915 (US); VERBOOMEN, Jason, Andrew, Appleton, Wisconsin 54913 (US); SCHOON, Bradley, William, Oshkosh, Wisconsin 54904 (US)
(74) Representative: Davies, Christopher Robert
(86) International application number: PCT/IB2013/060825
(87) International publication number: WO 2014/102640

(56) References cited:
- EP-A1- 1 779 827
- WO-A1-00/37007
- WO-A1-02/00152
- WO-A1-2008/056278
- WO-A1-2012/009316
- WO-A1-2014/076594
- WO-A2-00/35395
- WO-A2-01/87752
- US-A1- 2011 173 796
- US-A1- 2012 238 988

## Description

### BACKGROUND

Pant-like disposable undergarments for absorbing human discharges can appear similar in size and shape to regular cloth underwear which is designed to be laundered and reused. A disposable absorbent undergarment is intended to be worn by persons, including infants, toddlers, or adults, and is designed for a single or temporary use and is meant to be disposed of after being used once instead of being laundered or dry cleaned for re-use. Some examples of disposable undergarments include infant diapers, training pants, adult incontinence garments, feminine pants, etc.

Some pant-like disposable absorbent undergarments manufactured today resemble regular cloth underwear in that they have a waist opening and a pair of leg openings. Such pant-like disposable absorbent undergarments can be pulled up around the torso of a wearer in a similar fashion as regular cloth underwear. Still other pant-like disposable absorbent undergarments have an open or flat configuration and are designed to be placed adjacent to a wearer's torso and then rely upon one or more attachment tabs or fasteners to secure the undergarment around the wearer's torso. This design is beneficial for bed bound users who may be immobile or for children who need assistance in securing the undergarment in place. Still other adjustable, pant-like absorbent undergarments contain attachment means for opening and closing the waist opening after the undergarment has been positioned around the wearer's torso. This type of adjustable undergarment has an advantage in that the wearer does not have to remove outer clothing in order to check the status of the undergarment or to remove the undergarment from their body.

One example of such an adjustable, pant-like disposable absorbent undergarment includes a pair of lines of weakness that a user must break to enable adjusting the fit of the undergarment. The lines of weakness usually extend from the waist opening to one of the leg openings and are designed to be broken either prior to positioning the undergarment around the user's torso or while the undergarment is already positioned around the wearer's torso. A pair of fastener assemblies is then utilized to refasten the undergarment so that it is snug about the wearer's torso.

It has been found that a major portion of each of the lines of weakness is visually hidden and some users cannot see them and thereby do not know that they are present. In addition, each line of weakness may be ergonomically hard to tear open by older adults, some of who may be suffering from arthritis. In addition, options that require tearing often indicate to users that the product is damaged or of poor quality when torn.

An adjustable, pant-like disposable absorbent undergarment that includes a fully severed front panel and more readily apparent and more easily accessible fastener mechanisms is needed. Typically, however, the types of consumer goods mentioned above are manufactured on a continuous basis on large scale manufacturing lines. Usually, various raw products or components are formed on, or integrated into, a continuous stream of material, which often includes a web of material that moves in a machine direction through and along the line. As such, it is important to maintain the integrity of the stream of material or web during the process so as to avoid costly downtime. In general, the web is pushed or pulled along the line, so as to put the web in tension. Accordingly, the formation of a fully severed panel, especially along a cross-direction, can increase the risk of breakage. Therefore, it is desirable to maintain the tensile strength of the stream of materials or web as it passes through the process.

Therefore, there is a need to provide a method and apparatus for manufacturing pant-like disposable absorbent undergarment that includes a fully or partially severed front panel without causing a problem with the integrity of the stream of material or web during the process.

### SUMMARY

Generally, a method and apparatus for producing a pre-fastened adjustable pant-like disposable absorbent undergarment including a fully or partially severed front body panel is disclosed. The method includes first providing a discrete undergarment moving longitudinally in a machine direction, the discrete undergarment including a front panel, or first panel, a rear panel or second panel attached to the first panel at side seams, an absorbent assembly attached between the first panel and second panel, a waist opening and a pair of leg openings. The first panel and the second panel are separated in a z-direction to allow for a transport support structure to be passed through the waist opening and the pair of leg openings. While on the transport support structure, the first panel is cut to define a first portion of the front panel and a second portion of the front panel. Finally, the first portion of the front panel and the second portion of the front panel may be joined together with a fastener assembly that connects the first portion and the second portion.

The front and back body panels may be separated by any known means to those skilled in the art. For example, separating the first panel and the second panel may be done using both an upper and lower opposing vacuum devices. In this embodiment, an upper vacuum device pulls the first panel up in the z-direction while the lower vacuum device pulls the second panel down in the z-direction. In another embodiment, an upper vacuum device pulls the first panel up in the z-direction while gravity pulls the second panel down in the z-direction. In another embodiment, a lower vacuum device pulls the second panel down in the z-direction while compressed air from the lower vacuum device pushes the first panel up in the z-direction, and combinations thereof. In each case, the front and back panel separate causing the leg openings and waist opening to be exposed.

In one embodiment, the transport support structure includes a pair of support bars or skis. Each support bar extends through one of the leg openings and waist opening to support the front panel of the discrete undergarment and the discrete undergarment travels longitudinally in the machine direction. The support bars may extend along the length of the process necessary to sever the front panel and place the fastener assemblies on the discrete undergarment.

In some embodiments, the discrete undergarment is also passed through a folding apparatus. In one embodiment, folding the discrete undergarment includes stretching the discrete undergarment to separate the first panel and second panel in the z-direction, inserting a folding wheel into the middle of the pant, and folding the side seams into the discrete undergarment. In another embodiment, folding the discrete undergarment includes bending the transport support structure in the z-direction at the exit, folding the absorbent assembly over the first panel, and folding the side seams over the absorbent assembly. In another embodiment, folding the discrete undergarment includes twisting the transport support structure, folding the absorbent assembly over the first panel, and folding the side seams over the absorbent assembly.

Optionally, after the fastener assembly is attached, a side seam bond detector is used to detect the presence of the side seam as the discrete undergarment is being transferred down the transport support structure. The side seam can be detected since while on the transport support structure, the undergarment is stretched so that the front panel is separated from the back panel. If no side seam, or an unacceptable side seam is detected, the discrete undergarment is discarded. If acceptable, the discrete undergarment continues down the manufacturing line.

In some embodiments, the discrete undergarment is then passed through a folding apparatus. In one embodiment, folding the discrete undergarment includes stretching the discrete undergarment to separate the first panel and second panel in the z-direction, inserting a folding wheel into the middle of the discrete undergarment, and folding the side seams into the discrete undergarment. In another embodiment, folding the discrete undergarment includes bending the transport support structure in the z-direction at the exit, folding the absorbent assembly over the first panel, and folding the side seams over the absorbent assembly. In another embodiment, folding the discrete undergarment includes twisting the transport support structure, folding the absorbent assembly over the first panel, and folding the side seams over the absorbent assembly.

These features will be described in greater detail herein. Further, it is to be understood that both the foregoing general description and the following detailed description are exemplary and are intended to provide further explanation of the invention claimed.

### BRIEF DESCRIPTION

Figure 1 depicts a front perspective view of one example of a garment, the garment shown in a pre-fastened, pant-like configuration.
Figure 2 depicts a front perspective view of one example of a garment, the garment shown in an unfastened, pant-like configuration.
Figure 3 depicts a plan view of the garment of Figure 1, the garment shown in an unfastened, laid-open, relaxed configuration.
Figure 4 depicts an apparatus for producing a pre-fastened adjustable pant-like disposable absorbent undergarment including a fully severed front body panel.
Figure 5 depicts a top view of the transport support structure for use with the apparatus of Figure 4.
Figure 6 depicts a top view of a folding station for use with the apparatus of Figure 4.

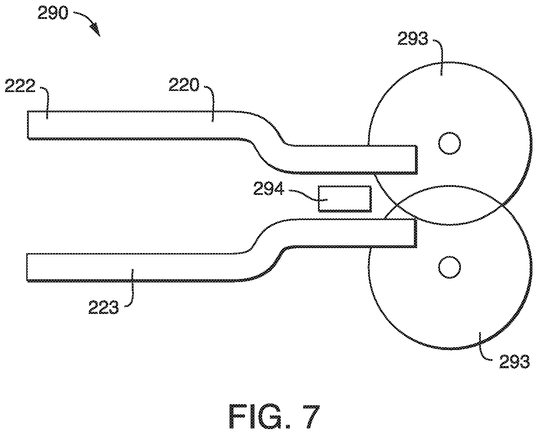

Figure 7 depicts a top view of an alternative folding station for use with the apparatus of Figure 4.
Figure 8 depicts a top view of another alternative folding station for use with the apparatus of Figure 4.

### DETAILED DESCRIPTION

Reference to the Figures shall be made in describing various embodiments. It should be noted that the embodiments depicted in the Figures and described herein are merely representative examples. The various embodiments are suitable for use in conjunction with disposable absorbent undergarments such as refastenable adult incontinence underwear, pre-fastened disposable diapers, refastenable disposable training pants or swim pants, refastenable disposable enuresis garments, and the like. For illustration purposes, various embodiments shall be described in conjunction with refastenable incontinence or enuresis underwear.

Within the context of this specification, each term or phrase below will include the following meaning or meanings.

The term "body side" should not be interpreted to mean in contact with the body of the user, but rather simply means the side that would face toward the body of the user when the garment is applied to the user, regardless of whether the absorbent garment is actually being worn by the user and regardless of whether there are or may be intervening layers between the component and the body of the user. Likewise, the term "garment side" should not be interpreted to mean in contact with the garments of the user, but rather simply means the side that faces away from the body of the user when the garment is applied to the user, and therefore toward any outer garments that may be worn by the user, regardless of whether the absorbent garment is actually being worn by a user, regardless of whether any such outer garments are actually worn and regardless of whether there may be intervening layers between the component and any outer garment.

The term "machine-direction" means the direction of flow as the various members and webs progress along the fabrication line and process. It should be understood that various separate members or webs can each be traveling in a machine direction, but with the various machine directions not necessarily being parallel or oriented in the same direction. For example, one web may be traveling along a first machine direction, which is substantially perpendicular to the travel of another web in a second machine direction.

The term "cross-direction" means the direction substantially perpendicular to the machine direction.

The term "z-direction" means the direction substantially perpendicular to the plane formed by the machine and cross-machine direction.

The term "downstream" means that one item is positioned more closely to the output or finished product end of the machine and/or process relative to another item. Conversely, the term "upstream" means that an item is positioned more closely to the input end of the machine or process relative to another item. For example, the output end is downstream of the input end, and vice versa, the input end is upstream of the output end.

The term "disposable absorbent undergarment" as used herein is an article that is intended to be worn by persons, including infants, toddlers or adults, which is designed for a single or temporary use and is meant to be disposed of after being used once instead of being laundered or dry cleaned for re-use.

The term "attached" refers to the joining, adhering, bonding, connecting, or the like, of two elements. Two elements will be considered to be attached together when they are attached directly to one another or indirectly to one another, such as when each is directly attached to intermediate elements.

The term "disposable" refers to articles which are designed to be discarded after a limited use rather than being laundered or otherwise restored for reuse.

The term "elastomeric" refers to a material or composite which can be elongated by at least 50% of its relaxed length and which will recover, upon release of the applied force, at least 20% of its elongation. It is generally preferred that the elastomeric material or composite be capable of being elongated by at least 100%, more preferably by at least 200%, of its relaxed length and recover, upon release of an applied force, at least 50% of its elongation.

The term "weakening" means to cause to lose strength, such that the area that is weakened is not as strong as the adjacent areas. For example, and without limitation, an area that is weakened may have a lesser tear or tensile strength as compared with the adjacent areas of the web, such that the web is more likely to be torn or broken along the area of weakness rather than the adjacent areas. In this way, the manufacturer can control the area of the web that will be broken, whether such breakage is performed by the end user or at a later time during the manufacturing or fabrication process.

The term "line of weakness" refers to any region or area of weakened material, preferably having a length and which may or may not have a defined width, and can include linear and non-linear patterns, such as curvilinear patterns of weakness, or other shapes, such as circles, rectangles, etc. The line of weakness can include a perforation or other series of cuts, a thinning, or breakage or separation of material, or a strip of a different kind of material bridging between adjacent portions of material, that is more easily torn or broken than the adjacent portions, and which allow the user or manufacturer to separate the adjacent portions along the line of weakness.

The terms "longitudinal" and "transverse" have their customary meaning, as indicated by the longitudinal and transverse axes depicted in the Figures. The longitudinal axis lies in the plane of the article and is generally parallel to a vertical plane that bisects a standing wearer into left and right body halves when the article is worn. The transverse axis lies in the plane of the article generally perpendicular to the longitudinal axis.

These terms may be defined with additional language in the remaining portions of the specification.

Generally, a method and apparatus for producing a pre-fastened adjustable pant-like disposable absorbent undergarment including a fully or partially severed front body panel is disclosed. The method includes first providing a discrete undergarment moving longitudinally in a machine direction, the discrete undergarment including a front panel, or first panel, a rear panel or second panel attached to the first panel at side seams, an absorbent assembly attached between the first panel and second panel, a waist opening and a pair of leg openings. The first panel and the second panel are separated in a z-direction to allow for a transport support structure to be passed through the waist opening and the pair of leg openings. While on the transport support structure, the first panel is cut to define a first portion of the front panel and a second portion of the front panel. Finally, the first portion of the front panel and the second portion of the front panel may be joined together with a fastener assembly that connects the first portion and the second portion.

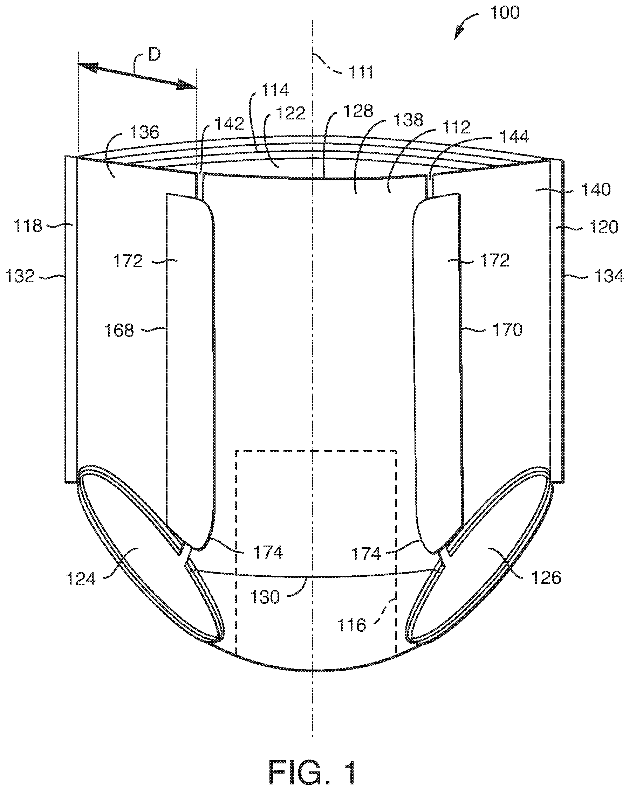

Referring to Figures 1-3, an exemplary adjustable pant-like disposable absorbent undergarment 100 having a longitudinal axis 111 made using the method described herein is shown. The adjustable pant-like disposable absorbent undergarment 100 is designed to absorb liquid, semi-solid and/or solid waste discharged from a human being. The adjustable pant-like disposable absorbent undergarment 100 is designed to absorb and/or retain one or more bodily discharges of waste material such as urine, perspiration, excrement, feces, menses, menstrual fluid, as well as other liquid and/or solid waste.

The adjustable pant-like disposable absorbent undergarment 100 includes a front body panel 112, a back body panel 114 and an absorbent assembly 116 secured to the front and back body panels, 112 and 114 respectively. The front and back body panels, 112 and 114 respectively, are joined together by a pair of seams 118 and 120 to form a waist opening 122 and a pair of leg openings 124 and 126.

The front and back panels 112 and 114 can be formed from a single piece of material or can be formed as a laminate consisting of two or more layers. The layers of the laminate can be of the same material or different material. In one embodiment, a laminate is formed from a first layer and a second layer. Sandwiched between the first and second layers are two or more elastic strands. Desirably, from two to about a hundred elastic strands can be utilized in the front and back panels 112 and 114 depending upon the overall size of each panel 112, 114. The elastic strands can be formed from LYCRA, or a similar material. LYCRA is a trademark of INVISTA (Wichita, KS). The diameter and/or cross-sectional configuration of the elastic strands, the decitex (weight in grams per 10,000 meters) of the elastic strands, and the tension imparted into the elastic strands can all be varied to suit one's particular product needs. The exact number of elastic strands that are utilized should be sufficient to ensure that the disposable absorbent undergarment snuggly conforms to the wearer's torso.

The elastic strands can be coated with an adhesive. By adhesively coating each of the elastic strands, instead of slot coating a major portion of the inner surface of at least one of the first and second layers, softer front and back panels 112 and 114 respectively, can be obtained. Wearers of disposable absorbent undergarments prefer a product that has a softer feel since it is more like underwear.

In other embodiments, the elastomeric nonwoven material comprises an elastomeric film sandwiched between two nonwoven facing layers. U.S. Patent No. 7,803,244 to Siqueira et al., hereby incorporated by reference, discloses particular examples of elastomeric nonwoven composites suitable for use in the adjustable undergarment described herein.

It should be noted that the front and back panels 112 and 114 can be formed from a breathable or a non-breathable material. Desirably, the front and back panels 112 and 114 are formed from a breathable material or a material that is treated or processed to be breathable. Spunbond and bonded carded webs are two breathable materials that work well as front and back panels 112 and 114 in disposable absorbent undergarments 100. Bonded carded webs are produced and commercially sold by a variety of vendors. Other materials that can be used to form the front and back panels 112 and 114 include woven and nonwoven materials formed from natural or synthetic fibers; polyolefins, such as polypropylene or polyethylene; thermoplastic films; as well as other materials known to those skilled in the art. A metallocene polypropylene works very well since it has a soft feel and can be easily ultrasonically bonded to itself.

The front body panel 112 includes a waist edge 128, a crotch edge 130 and a pair of side edges 132 and 134. In a three piece construction wherein an absorbent assembly 116 is secured between the front body panel 112 and the back body panel 114, the crotch edge 130 is well defined. In absorbent undergarments of a different construction, the crotch edge 130 can be an imaginary line transversely drawn between the pair of leg openings 124 and 126 at a location where one considers the front body panel 112 to end. The exact size and configuration of the front body panel 112 can vary to suit a wearer's particular needs. The front body panel 112 has a first side section 136, a middle section 138 and a second side section 140.

In one embodiment, the front body panel 112 is completely severed between both the first side section 136 and the middle section 138 and the second side section 140 and the middle section 138 creating a first fully severed region, or gap, 142 and a second fully severed region 144. The first side section 136 is aligned adjacent to the side edge 132 at its leading edge forming the seam 120 and the second side section 140 is aligned adjacent to the side edge 134 at its leading edge forming the seam 118. The middle section 138 has a first edge 152 and a second edge 154 and is located between the first and second side sections, 136 and 140 respectively. The terminal edge 150 of the first side section 136 is aligned adjacent to the first edge 152 of the middle section 138 and the terminal edge 150 of the second side section 140 is aligned adjacent to the second edge 154 of the middle section 138. As depicted, the middle section 138 is centrally located and is bifurcated by the longitudinal axis 111 at a midpoint of the front body panel 112. In other embodiments not illustrated, the front body panel 112 is weakened between both the first side section 136 and the middle section 138 and the second side section 140 and the middle section 138 creating a first line of weakness and a second line of weakness.

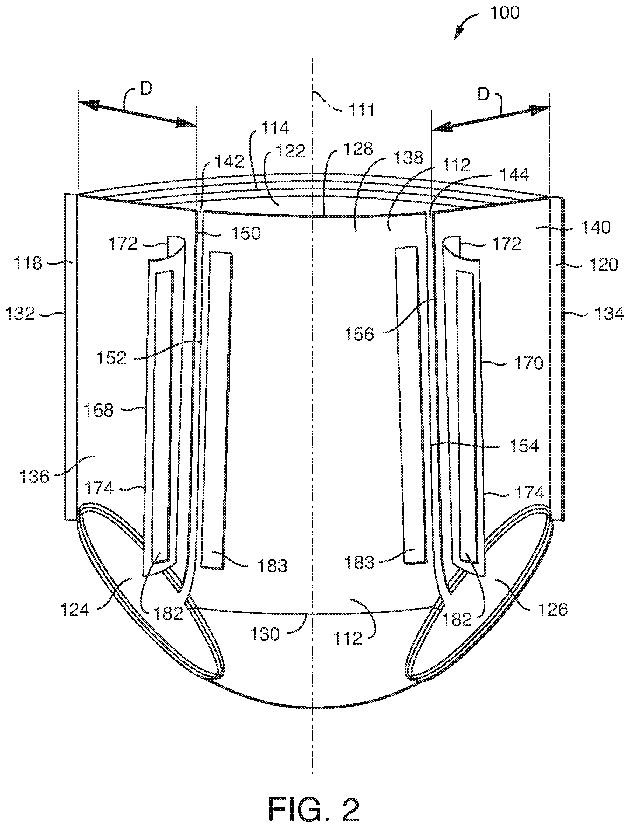

The first and second fully severed regions, 142 and 144, can be linear or non-linear in configuration. In Figures 2-3, the first and second fully severed region, 142 and 144, are shown having a linear or straight configuration. The first and second fully severed regions, 142 and 144, extend longitudinally from approximately the waist edge 128 down to approximately one of the leg openings 124 or 126. The first and second fully severed regions, 142 and 144, can be aligned parallel to the longitudinal axis 111 or be angled thereto. For example, the first and second fully severed regions, 142 and 144, can be tapered relative to the longitudinal axis 111, if desired. In addition, the fully severed regions, 142 and 144, can also be curved.

Still referring to Figures 1-3, each of the first and second fully severed regions, 142 and 144, extend from approximately the waist edge 128 of the front body panel 112 to one of the pair of leg openings 124 and 126. Another way of describing this is to say that the first and second fully severed regions, 142 and 144, extend from approximately the waist edge 128 of the front body panel 112 to approximately the crotch edge 130 of the front body panel 112.

Still referring to Figures 2-3, each of the first and second fully severed regions, 142 and 144, is shown being aligned parallel to one of the pair of seams 118 and 120. Such an arrangement provides for an aesthetically pleasing appearance to the front body panel 112.

Desirably, in the pre-fastened condition, the first side section 136 and the middle section 138 of front body panel 112 do not overlap, and the second side section 140 and the middle section 138 of the front body panel 112 do not overlap in the severed regions 142, 144. In one exemplary embodiment, a first gap may be formed in the first severed region 142 between the first side section 136 and the middle section 138 of the front body panel 112 in the pre-fastened condition. In this embodiment, a second gap may be formed in the first severed region 142 between the second side section 140 and the middle section 138 of the front body panel 112 in the pre-fastened condition. In another exemplary embodiment, a terminal or outer edge 150 of the first side section 136 abuts against the first edge 152 of the middle section 138 of the front body panel 112 in the pre-fastened condition. In this embodiment, a terminal or outer edge 150 of the second side section 140 abuts against the second edge 154 of the middle section 138 of the front body panel 112 in the pre-fastened condition. In another embodiment, a gap may be formed between one side section 136 and the middle section 138 and the other side section 140 and middle section 138 abut against each other. If a gap is formed between the side sections 136 and 140, and the middle section 138, the gap will desirably be less than 10 mm.

Referring again to Figures 2-3, the front body panel 112 further includes a pair of fastener assemblies 168 and 170. Each fastener assembly 168 and 170 includes a first portion 172 and a second portion 174. The first portion 172 of the fastener assembly 168 can be permanently secured to the first side section 136 of the front body panel 112 and the first portion 172 of the other fastener assembly 170 can be permanently secured to the second side section 140 of the front body panel 112.

In the embodiment shown in Figures 2-3, the first portions 172 of the fastener assemblies 168 and 170 can be securely attached using an adhesive, heat, pressure, a combination of heat and pressure, an ultrasonic bond, a chemical bond or by other means known to those skilled in the art.

Each of the second portions 174 of the fastener assemblies 168 and 170 are depicted as extending almost the entire length of the fully severed portions 142 and 144. This means at least 50% of the distance between the waist edge 128 and the leg opening 124 are covered by the first fastener assembly 168. Desirably, the first fastener assembly 168 and second fastener assembly 170 extend at least 65% of the distance between the waist edge 128 and the leg opening 124. More desirably, the first fastener assembly 168 and second fastener assembly 170 extend at least 75% of the distance between the waist edge 128 and the leg opening 124. Even more desirably, the first fastener assembly 168 and second fastener assembly 170 extend at least 85% of the distance between the waist edge 128 and the leg opening 124. Even more desirably, the first fastener assembly 168 and second fastener assembly 170 completely extend at least 95% of the distance between the waist edge 128 and the leg opening 124. The second portion 174 of the first fastener assembly 168 can bridge across the first fully severed region 142 and the second portion 174 of the other fastener assembly 170 can bridge across the second fully severed region 144. By extending substantially the entire length of and bridging the fully severed portions, 142 and 144, the fastener assemblies 168, 170 cover the fully severed portions 142, 144 and provide a more underwear like appearance. The second portion 174 of the fastener assemblies 168 and 170 can be releasably attached to the middle section 138 of the front body panel 112.

Alternatively, it should be evident to those skilled in the art that the first portion 172 of each of the fastener assemblies 168 and 170 could be permanently attached to the middle section 138. In this embodiment, the second portion 174 of the fastener assemblies 168 and 170 can be releasably attached to the side sections 136, 140 of the front body panel 112. In an alternative embodiment, both the first portion 172 and the second portion 174 may be releasably attached. The fastener assembly 168 may be constructed of a non-extensible or an elastomeric material.

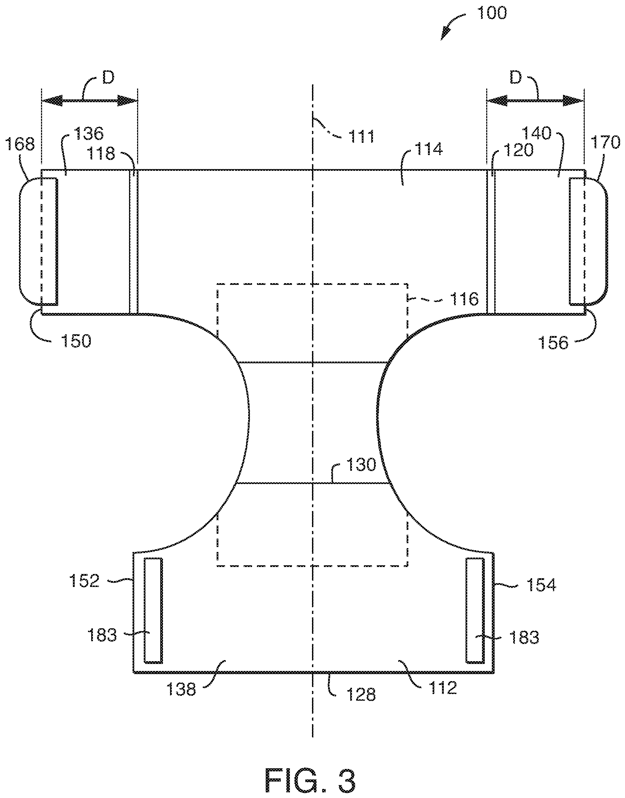

Referring now to Figures 2-3, each of the second portions 174 of the fastener assemblies 168 and 170 has an inner surface 180 that contains a fastener 182. The fastener 182 can be a mechanical fastener 182. In Figures 2 and 3, the mechanical fastener 182 is shown as a plurality of fine hooks, such as VELCRO hooks. VELCRO is a trademark of Velcro USA, Inc. (Manchester, NH). The hooks are designed to easily engage and be removed from a material wherein the material has a loose weave pattern or the fibers forming the material will allow the hooks to be attached to them. The mating material is commonly referred to as the loop member of a hook and loop fastener. The middle section 138 of the front body panel 112 may be formed of such a material. When the hooks engage into the middle section 138, a secure but releasable fastener 182 is formed. The hooks can be easily removed from the loop material by pulling the edge of the fastener 182 outward away from the middle section 138. Alternatively, as illustrated in Figures 2 and 3, a fastening component 183 such as a loop material or loose fibers that may be placed anywhere onto the middle section 138 to facilitate the fastening component 183. Therefore, hook and loop fasteners are referred to as being releasable and can be fastened and released several times. In alternative embodiments, the mechanical fastener 182 constructed of hooks may be placed on to the middle section 138 of the front body panel 112. In this embodiment, the fastening component 183 constructed of a loop material or loose fibers would be placed on the fastener assemblies 168, 170. Both the mechanical fastener 182 and the fastening component 183 may be integral or separately attached.

Referring again to Figures 1-3, one can see that Figure 1 depicts the pair of fastener assemblies 168 and 170 being securely fastened to the middle section 138 of the front body panel 112. If the wearer of the disposable absorbent undergarment 100 or a caregiver wishes to inspect the undergarment 100, he or she would open the pair of fastener assemblies 168 and 170 to the position shown in Figure 2. Since the first and fully severed portions, 142 and 144 are already broken, the middle section 138 of the disposable absorbent undergarment 100 can be easily moved outward away from the wearer's torso. The wearer can then inspect the absorbent assembly 116 to see if it needs to be changed. If so, the disposable absorbent undergarment 100 can be removed from about the wearer's torso and be replaced by another undergarment. If the absorbent assembly 116 is still capable of accepting additional body fluid, the middle section 138 is moved back against the wearer's torso and the pair of fastener assemblies 168 and 170 is refastened to the middle section 138.

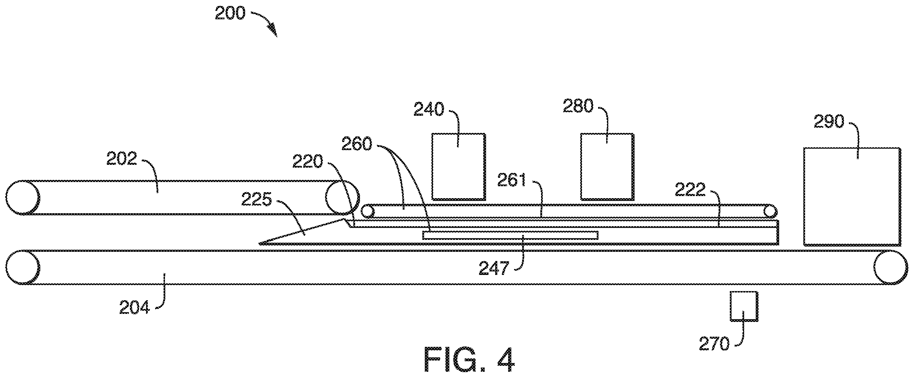

The horizontal distance to each of the first and second fully severed regions, 142 and 144, and corresponding first and second fastener assemblies, 168 and 170, as located relative to the pair of side seams 118 and 120 can also vary. A distance "D" is depicted in Figures 4-5 which represents the distance between each of the pair of seams 118 and 120 and the corresponding first and second fully severed regions, 142 and 144 respectively. One can increase the distance "D" by moving the first and second fully severed regions, 142 and 144 respectively, away from the pair of seams 118 and 120. When one increases the distance "D", one may find that it is easier for the wearer of the disposable absorbent undergarment 100 to visually see and identify the first and second fully severed regions, 142 and 144, when user looks down at the front body panel 112. In some embodiments, the first fastener assembly 168 and the second fastener assembly 170 are located near the midpoint between the side seams, 118 and 120, and the cross-sectional midpoint 111 of the front panel 112. Desirably, the first fastener assembly 168 and second fastener assembly 170 are located between 30% and 70% of the distance between the side seam, 118 and 120, and the cross-sectional midpoint 111 of the front panel 112. More desirably, the first fastener assembly 168 and second fastener assembly 170 are located between 35% and 45% of the distance between the side seams, 118 and 120, and the cross-sectional midpoint 111 of the front body panel 112. A manufacturer is free to vary the distance "D" to best suit the size and shape of a particular disposable absorbent undergarment 100 to make sure that the fastener 182 is on the front of the product rather than the side of the product. If the fully severed regions 142, 144 are not parallel to the side seams 118, 120, the midpoint of the fully severed regions 142, 144 in the longitudinal direction may be used to measure "D".

Placement near the midpoint between the side seam and the cross-sectional midpoint 111 of the front body panel 112 facilitates enhanced donning and removal when experiencing a reduced range of motion as well as locating the fastener 182 in a location which is easier for the consumer to see.

The placement of the fastener assemblies 168 and 170 in the front of the product optimizes the ability of the user to fasten the product, while minimizing potential pop-opens during use due to movement of the legs of the user. The placement of the fasteners 182 can be measured in both an unstretched (out of the bag) state or stretched (as in use).

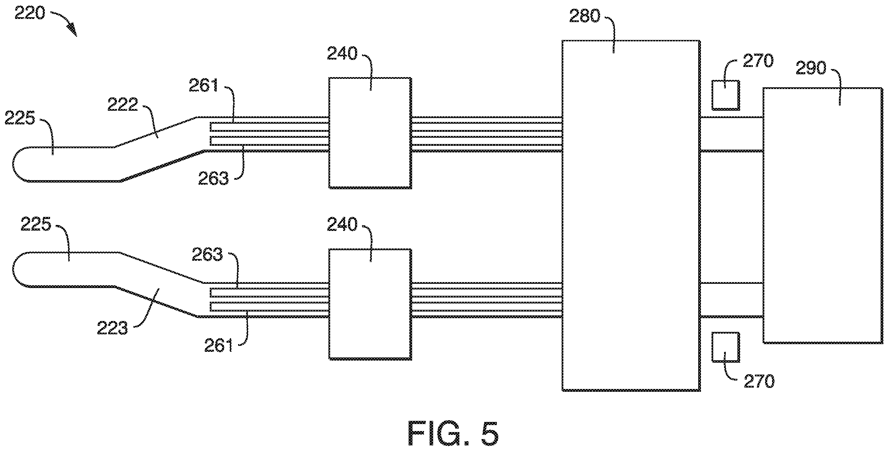

Referring to Figures 4 and 5, a method and apparatus 200 for producing the discrete pre-fastened adjustable pant-like disposable absorbent undergarment 100 described above is illustrated. The discrete undergarment 100 moves in a machine direction along a process line. In one embodiment as described above, the discrete undergarment 100 includes a front body panel 112 and bonded to a back body panel web 114 at side seams 118, 120, wherein the back body panel web 114 is positioned over the front body panel 112 with and absorbent assembly 116 joining or bridging between the front body panel 112 and back panel 114.

Once the discrete undergarment 100 is provided, the discreet undergarment 100 is opened up. In other words, the front panel 112 and the back panel 114 are separated from each other in a z-direction. The front and back body panels 112, 114 may be separated by any know means to those skilled in the art with a panel separating device. For example, as illustrated in Figure 4, separating the front panel 112 and the back panel 114 may done using both an upper and lower opposing vacuum devices, 202 and 204. In this embodiment, an upper vacuum device 202 pulls the front panel 112 up in the z-direction while the lower vacuum device 204 pulls the back panel 114 down in the z-direction. In another embodiment, an upper vacuum device 202 pulls the front panel 112 up in the z-direction while gravity pulls the back panel 114 down in the z-direction. In another embodiment, a lower vacuum device 204 pulls the back panel 114 down in the z-direction while compressed air from the lower vacuum device 204 pushes the front panel 112 up in the z-direction. Combinations of the above embodiments may also be used. In each case, the front and back panel 112, 114 separate causing the leg openings 124, 126 and waist opening 128 to be exposed.

Once the front panel 112 and back panel 114 have been separated to expose the leg openings 124, 126 and waist opening 128, the discrete undergarment 100 is placed onto a transport support structure 220. In one embodiment, the transport support structure 220 includes a pair of support bars or skis, 222 and 223. Each support bar 222, 223 extends through one of the leg openings 124, 126 and waist opening 128 to support the front panel 112 of the discrete undergarment 100 as the discrete undergarment 100 travels longitudinally in the machine direction. The support bars 222, 223 may extend along the length of the process necessary to sever or cut the front panel 112 and place the fastener assemblies 168, 170 on the discrete undergarment 100.

Desirably, the transfer support structure 220 has a tapered front edge 225 to allow for easier placement through the leg openings 124,126 and waist opening 128 on the discrete undergarment 100. In exemplary embodiments, the tapered front edge 225 of the transfer support structure 220 widens in the cross machine direction after the discrete undergarment 100 is placed onto the transport support structure 220, stretching the front panel 112 and back panel 114 on the discrete undergarment 100 in the cross-machine direction. Stretching the front panel 112 of the discrete undergarment 100 allows for easier placement onto the support bars 222, 223 and more efficient cutting of an elastomeric panel as it is transported through the process.

While on the transport support structure 220, the discrete undergarment 100 is passed through a panel cutting station 240. In one desirable embodiment, the panel cutting station 240 is configured as a perforator having a knife roll and an anvil roll. Alternatively, the panel cutting station 240 can be configured with a flex knife, laser, water jet, or other types of cutters known to those skilled in the art. In other alternative embodiments, the panel cutting station 240 can comprise a device for applying heat, thermal energy or ultrasonic energy to the web so as to cut it at specific locations. In other preferred embodiments, the panel cutting station 240 can include a chemical applicator that applies various chemicals, including for example water, to the web to cut it at specific locations. In yet another alternative embodiment, the apparatus applies a speed differential to the web so as to cut the web. Of course, it should be understood that the panel cutting station 240 can also be configured from combinations of one or more of the above-referenced devices.

In an exemplary embodiment, the panel cutting station 240 completely separates a first portion of the front body panel 112 and a middle portion of the front body panel 112 and completely separates the middle portion of the front body panel and a third portion of the front body panel 112. In another embodiment, the panel cutting station 240 cuts the front panel 112 to form a line of weakness between the first portion of the front body panel 112 and the middle portion of the front body panel 112. This line of weakness could include a series of intermittent perforations. Alternatively, the line of weakness could include scoring the web, or cutting a depth into the material without breaking the material, to provide a weakened area. In these embodiments, the front body panel 112 is not completely severed, but provides a weakened area that a user of the disposable absorbent undergarment 100 may easily break to readjust the garment for a better fit. Alternatively, the line of weakness may be broken further downstream in any manner known to those skilled in the art.

As the front body panel 112 of the discrete undergarment 100 is cut until the fastener is placed, stability of the discrete undergarment 100 is important. Accordingly, the transport support structure 220 may also contain a panel stability module 260. In one embodiment the panel stability module 260 may include at least one belt or chain 261, 263 traveling in the machine direction aligned on each of the support bars 222, 223 to secure the front body panel 112 between the belt 261 and support bar 222. In this embodiment, when the front body panel 112 is cut, the belt or chain 261, 263 maintains the contact with the front body panel 112 to ensure placement against the support bars 222, 223.

In one embodiment, as illustrated in Figure 5, the transport support structure 220 may include a pair of belts 261, 263 traveling in the machine direction aligned over each of the support bars 222, 223 to secure the front body panel 112 between the pair of belts 261, 263 and the support bar 222. In this embodiment, it may be desirable to cut the front body panel 112 between the two belts 261, 263 for further stability control.

The transport support structure 220 may also contain other mechanisms to help maintain panel stability. For example, the lower conveyor may be a vacuum device 202 to help maintain stability of the back body panel 114 as it is processed on the transport support structure 220. In another embodiment, a vacuum device 202 underneath the support bars 222, 223 may help support the front body panel 112. Furthermore, compressed air may blow from above the transport support structure 220 to help maintain the front body panel 112 on the support bars 222, 223.

While on the transport support structure 220, the discrete undergarment 100 is then passed through a fastener attachment station 280. The fastener attachment station 280 attaches a fastener assemblies 168, 170 over the cut or severed area of the front panel 112 together with a fastener assemblies 168, 170. Examples of way to attach a fastener assemblies 168, 170 include use of a cut and place module, slip cut module, or indexing conveyor to apply the fastener assemblies 168, 170 over the severed front panel 112 of the discrete undergarment 100.

The fastener assemblies 168, 170 will help maintain the integrity of the discrete undergarment 100 as it continues through the remainder of the process so the discrete undergarment 100 may now be transferred off of the transport support structure 220.

Various methods and apparatus for applying fastener members thereto are disclosed in U.S. Patent No. 6,730,188 entitled "Method and Apparatus For Assembling Refastenable Absorbent Garments," U.S. Patent No. 6,743,321 entitled "Method and Apparatus For Assembling Refastenable Absorbent Garments," U.S. Patent No. 6,686,626 entitled "Method and Apparatus For Assembling Refastenable Absorbent Garments," U.S. Patent No. 6,682,626 entitled "Method and Apparatus For Assembling Refastenable Absorbent Garments," U.S. Patent No. 6,712,922 entitled "Multiple Component Web," U.S. Patent No. 6,730,188 entitled "Method of Assembling Personal Care Absorbent Article," U.S. Patent No. 6,783,487, entitled "Pant-Type Personal Care Articles, and Methods of Making and Using Such Personal Care Articles," U.S. Patent 6,454,888 entitled "Methods of Changing Size of Pant-Type Personal Care Articles Outputted from a Manufacturing Process," the entire disclosures of which are hereby incorporated by reference.

Optionally, a side seam bond detector 270 is used to detect the presence of the side seam 118 as the discrete undergarment 100 is being transferred down the transport support structure 220. The side seam 118 can be detected since while on the transport support structure 220, the discrete undergarment 100 is stretched so that the front body panel 112 is separated from the back body panel 114. If no side seam 118 is present, or an unacceptable side seam 118 is detected, the discrete undergarment 100 is discarded. If acceptable, the discrete undergarment 100 continues to be processed down the manufacturing line.

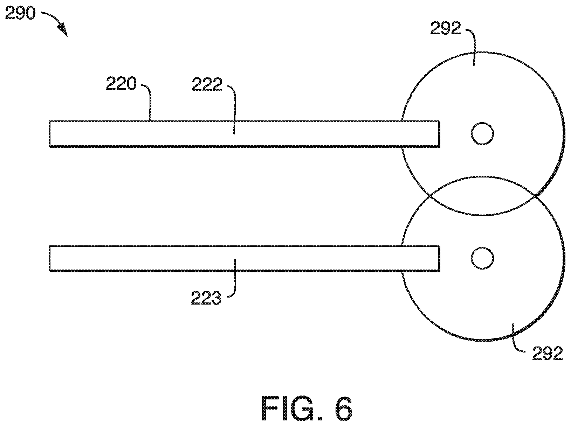

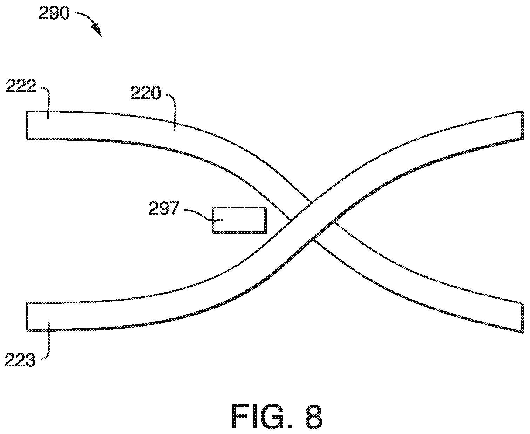

In some embodiments, the discrete undergarment 110 is then passed through a folding apparatus 290. In one embodiment as illustrated in Figure 6, the folding apparatus 290 will fold the discrete undergarment 100 by stretching the discrete undergarment to separate the front panel 112 and back panel 114 in the z-direction, and inserting a folding wheel 292 into the middle of the discrete undergarment 100, thereby folding the side seams 118, 120 into the discrete undergarment 100. In this embodiment, the front body panel 112 and back body panel 114 are already separated while traveling on the transport support structure 220 enabling this fold to be done efficiently. In another embodiment illustrated in Figure 8, the folding apparatus 290 will fold the discrete undergarment 100 by bending the transport support structure 220 vertically in the z-direction at the exit, folding the absorbent assembly 168 over the front body panel 112 with a folding ski 294, and folding the side seams 118,120 over the absorbent assembly 168 with folding wheels 292. In another embodiment illustrated in Figure 8, folding the discrete undergarment 100 includes twisting the transport support structure 220 to bring the side seams 118, 120 of the discrete undergarment 100 together, and then folding the absorbent assembly 168 over the front body panel 112 with a folding ski 294.

It should be understood that various other embodiments, modifications, and equivalents to the embodiments of the absorbent article described herein which, after reading the description herein, may suggest themselves to those skilled in the art without departing from the scope and spirit of the present claims.

## Claims

1. A method of producing pre-fastened disposable undergarments, comprising:
a. providing a discrete undergarment moving longitudinally in a machine direction, the discrete undergarment including a first panel, a second panel attached to the first panel at side seams, an absorbent assembly attached between the first panel and second panel, a waist opening and a pair of leg openings;
b. separating the first panel and the second panel in a z-direction;
c. passing a transport support structure through the waist opening and the pair of leg openings;
d. cutting the first panel to define a first portion of the front panel and a second portion of the front panel; and
e. bridging the first portion of the front panel and the second portion of the front panel together with a fastener assembly that connects the first portion and the second portion.

2. The method of claim 1 wherein cutting the first panel comprises fully severing the first panel.

3. The method of claim 1 or 2 wherein the transport support structure comprises a pair of bars extending through the waist opening and the pair of leg openings to support the first panel and a lower conveyor to support the second panel.

4. The method of any of the preceding claims wherein the transport support structure comprises a tapered front end.

5. The method of any of the preceding claims wherein the transport support structure may further comprise at least one belt traveling in the machine direction to secure the front panel to at least one bar of the pair of bars.

6. The method of any of the preceding claims wherein the transport support structure may further comprise a vacuum device to secure the front panel to at least one bar of the pair of bars.

7. The method of any of the preceding claims further comprising stretching the adjustable refastenable undergarment in a cross-machine direction.

8. The method of any of the preceding claims wherein separating the first panel and the second panel may be selected from upper and lower opposing vacuum devices, an upper vacuum device, a lower vacuum device with compressed air, and combinations thereof.

9. The method of any of the preceding claims wherein stretching the undergarment comprises widening the transport support structure in the cross-machine direction.

10. The method of any of the preceding claims further comprising folding the discrete undergarment.

11. The method of any of the preceding claims wherein folding the discrete undergarment comprises stretching the discrete undergarment to separate the first panel and second panel in the z-direction, inserting a folding wheel into the middle of the discrete undergarment, and folding the side seams into the discrete undergarment.

12. The method of any of the preceding claims wherein folding the discrete undergarment comprises bending the transport support structure in the z-direction at the exit, folding the absorbent assembly over the first panel, and folding the side seams over the absorbent assembly.

13. The method of any of the preceding claims wherein folding the discrete undergarment comprises twisting the transport support structure, folding the absorbent assembly over the first panel, and folding the side seams over the absorbent assembly.

14. The method of any of the preceding claims further comprising detecting side seam bond presence as the discrete undergarment is being transferred down the transport support structure.

15. An apparatus for producing a pre-fastened adjustable pant-like disposable absorbent undergarment comprising:
a. a conveyor system having an upper conveyor and lower conveyor to provide a discrete undergarment moving longitudinally in a machine direction, the discrete undergarment including a first panel, a second panel attached to the first panel at side seams, an absorbent assembly attached between the first panel and second panel, a waist opening and a pair of leg openings;
b. a panel separation device;
c. a transport support device;
d. a panel cutting station for cutting the first panel to define a first portion of the front panel and a second portion of the front panel; and
e. a fastener assembly attachment station for bridging the first portion of the front panel and the second portion of the front panel.

## Patentansprüche

1. Verfahren zur Herstellung vorbefestigter wegwerfbarer Unterwäsche, umfassend:
a. das Bereitstellen eines einzelnen Stücks Unterwäsche, das sich der Länge nach in einer Maschinenrichtung bewegt, wobei das einzelne Stück Unterwäsche eine erste Stoffbahn, eine zweite Stoffbahn, die an der ersten Stoffbahn an den Seitennähten befestigt ist, eine absorptionsfähige Anordnung, die zwischen der ersten Stoffbahn und der zweiten Stoffbahn befestigt ist, eine Taillenöffnung und ein Paar Beinöffnungen enthält;
b. das Trennen der ersten Stoffbahn und der zweiten Stoffbahn in einer z-Richtung;
c. das Hindurchführen einer Transportstützstruktur durch die Taillenöffnung und das Paar Beinöffnungen;
d. das Schneiden der ersten Stoffbahn, um einen ersten Abschnitt der vorderen Stoffbahn und einen zweiten Abschnitt der vorderen Stoffbahn zu definieren; und
e. das zusammen Verbrücken des ersten Abschnitts der vorderen Stoffbahn und des zweiten Abschnitts der vorderen Stoffbahn mit einer Verschlussanordnung, die den ersten Abschnitt und den zweiten Abschnitt verbindet.

2. Verfahren nach Anspruch 1, wobei das Schneiden der ersten Stoffbahn das vollständige Trennen der ersten Stoffbahn umfasst.

3. Verfahren nach Anspruch 1 oder 2, wobei die Transportstützstruktur ein Paar Riegel, die sich durch die Taillenöffnung und das Paar Beinöffnungen erstrecken, um die erste Stoffbahn zu stützen, und einen unteren Förderer zum Stützen der zweiten Stoffbahn umfasst.

4. Verfahren nach einem der vorhergehenden Ansprüche, wobei die Transportstützstruktur ein sich verjüngendes vorderes Ende umfasst.

5. Verfahren nach einem der vorhergehenden Ansprüche, wobei die Transportstützstruktur ferner mindestens ein Band, das sich in Maschinenrichtung bewegt, zum Befestigen der vorderen Stoffbahn an mindestens einen Riegel des Paars von Riegeln umfasst.

6. Verfahren nach einem der vorhergehenden Ansprüche, wobei die Transportstützstruktur ferner eine Vakuumvorrichtung zum Befestigen der vorderen Stoffbahn an mindestens einen Riegel des Paars von Riegeln umfassen kann.

7. Verfahren nach einem der vorhergehenden Ansprüche, ferner das Strecken des verstellbaren, erneut befestigungsfähigen Stücks Unterwäsche in einer Quermaschinenrichtung umfassend.

8. Verfahren nach einem der vorhergehenden Ansprüche, wobei das Trennen der ersten Stoffbahn und der zweiten Stoffbahn unter oberen und unteren entgegengesetzten Vakuumvorrichtungen, einer oberen Vakuumvorrichtung, einer unteren Vakuumvorrichtung mit Druckluft, und Kombinationen davon ausgewählt werden kann.

9. Verfahren nach einem der vorhergehenden Ansprüche, wobei das Strecken des Stücks Unterwäsche das Erweitern der Transportstützstruktur in der Quermaschinenrichtung umfasst.

10. Verfahren nach einem der vorhergehenden Ansprüche, ferner das Falten des einzelnen Stücks Unterwäsche umfassend.

11. Verfahren nach einem der vorhergehenden Ansprüche, wobei das Falten des einzelnen Stücks Unterwäsche das Strecken des einzelnen Stücks Unterwäsche zum Trennen der ersten Stoffbahn und zweiten Stoffbahn der z-Richtung, das Einfügen eines Falzrads in die Mitte des einzelnen Stücks Unterwäsche und das Falten der Seitennähte zu dem einzelnen Stück Unterwäsche umfasst.

12. Verfahren nach einem der vorhergehenden Ansprüche, wobei das Falten des einzelnen Stücks Unterwäsche das Biegen der Transportstützstruktur in der z-Richtung am Ausgang, das Falten der absorptionsfähigen Anordnung über die erste Stoffbahn und das Falten der Seitennähte über die absorptionsfähige Anordnung umfasst.

13. Verfahren nach einem der vorhergehenden Ansprüche, wobei das Falten des einzelnen Stücks Unterwäsche das Verdrehen der Transportstützstruktur, das Falten der absorptionsfähigen Anordnung über die erste Stoffbahn und das Falten der Seitennähte über die absorptionsfähige Stoffbahn umfasst.

14. Verfahren nach einem der vorhergehenden Ansprüche, ferner das Erfassen des Vorliegens einer Seitennahtbindung umfassend, währen das einzelne Stück Unterwäsche an der Transportstützstruktur herunter übertragen wird.

15. Vorrichtung zur Herstellung eines vorbefestigten, einstellbaren, unterhosenähnlichen, wegwerfbaren, absorptionsfähigen Stücks Unterwäsche, umfassend:
a) ein Fördersystem, das einen oberen Förderer und einen unteren Förderer aufweist, um ein einzelnes Stück Unterwäsche bereitzustellen, das sich der Länge nach in einer Maschinenrichtung bewegt, wobei das einzelne Stück Unterwäsche eine erste Stoffbahn, eine zweite Stoffbahn, die an der ersten Stoffbahn an Seitennähten befestigt ist, eine absorptionsfähige Anordnung, die zwischen der ersten Stoffbahn und der zweiten Stoffbahn befestigt ist, eine Taillenöffnung und ein Paar Beinöffnungen enthält;
b) eine stoffbahntrennende Vorrichtung;
c) eine Transportstützvorrichtung;
d) eine Stoffbahnschneidestation zum Schneiden der ersten Stoffbahn, um einen ersten Abschnitt der vorderen Stoffbahn und einen zweiten Abschnitt der vorderen Stoffbahn zu definieren; und
e) eine Verschlussanordnungsbefestigungsstation zum Überbrücken des ersten Abschnitts der vorderen Stoffbahn und des zweiten Abschnitts der vorderen Stoffbahn.

## Revendications

1. Procédé pour produire des sous-vêtements jetables pré-fixés, comprenant :
a. la fourniture d'un sous-vêtement discret se déplaçant longitudinalement dans un sens machine, le sous-vêtement discret comportant un premier panneau, un deuxième panneau fixé au premier panneau au niveau de coutures latérales, un assemblage absorbant étant fixé entre le premier panneau et le deuxième panneau, une ouverture de ceinture et une paire d'ouvertures de jambes ;
b. la séparation du premier panneau et du deuxième panneau dans une direction z;
c. le passage d'une structure de support de transport à travers l'ouverture de taille et la paire d'ouvertures de jambes ;
d. la découpe du premier panneau pour définir une première partie du panneau avant et une deuxième partie du panneau avant ; et
e. la liaison de la première partie du panneau avant et de la deuxième partie du panneau avant conjointement avec un assemblage de fixation qui raccorde la première partie et la deuxième partie.

2. Procédé selon la revendication 1, dans lequel la découpe du premier panneau comprend la section complète du premier panneau.

3. Procédé selon la revendication 1 ou 2 dans lequel la structure de support de transport comprend une paire de barres s'étendant à travers l'ouverture de ceinture et la paire d'ouvertures de jambes pour supporter le premier panneau et un convoyeur inférieur pour supporter le deuxième panneau.

4. Procédé selon l'une quelconque des revendications précédentes dans lequel la structure de support de transport comprend une extrémité avant rétrécie.

5. Procédé selon l'une quelconque des revendications précédentes dans lequel la structure de support de transport peut comprendre en outre au moins une courroie se déplaçant dans le sens machine pour fixer le panneau avant à au moins une barre de la paire de barres.

6. Procédé selon l'une quelconque des revendications précédentes dans lequel la structure de support de transport peut comprendre en outre un dispositif de vide pour fixer le panneau avant à au moins une barre de la paire de barres.

7. Procédé selon l'une quelconque des revendications précédentes comprenant en outre l'étirement du sous-vêtement repositionnable ajustable dans un sens travers.

8. Procédé selon l'une quelconque des revendications précédentes, dans lequel la séparation du premier panneau et du deuxième panneau peut être choisie parmi des dispositifs de vide opposés supérieur et inférieur, un dispositif de vide supérieur, un dispositif de vide inférieur avec de l'air comprimé, et des combinaisons de ceux-ci.

9. Procédé selon l'une quelconque des revendications précédentes, dans lequel l'étirement du sous-vêtement comprend l'élargissement de la structure de support de transport dans le sens travers.

10. Procédé selon l'une quelconque des revendications précédentes, comprenant en outre le pliage du sous-vêtement discret.

11. Procédé selon l'une quelconque des revendications précédentes dans lequel le sous-vêtement discret comprend l'étirement du sous-vêtement discret pour séparer le premier panneau et le deuxième panneau dans la direction z, l'insertion d'une roue de pliage dans le milieu du sous-vêtement discret, et le pliage des coutures latérales dans le sous-vêtement discret.

12. Procédé selon l'une quelconque des revendications précédentes dans lequel le pliage du sous-vêtement discret comprend la courbure de la structure de support de transport dans la direction z à la sortie, le pliage de l'assemblage absorbant sur le premier panneau, et le pliage des coutures latérales sur l'assemblage absorbant.

13. Procédé selon l'une quelconque des revendications précédentes dans lequel le pliage du sous-vêtement discret comprend la torsion de la structure de support de transport, le pliage de l'assemblage absorbant sur le premier panneau, et le pliage des coutures latérales sur l'assemblage absorbant.

14. Procédé selon l'une quelconque des revendications comprenant en outre la détection de la présence d'une liaison de couture latérale lorsque le sous-vêtement discret est transféré vers la structure de support de transport.

15. Appareil pour produire des sous-vêtements jetables de type culottes pré-fixés, ajustables, comprenant :
a. un système convoyeur ayant un convoyeur supérieur et un convoyeur inférieur pour fournir un déplacement du sous-vêtement discret longitudinalement dans un sens machine, le sous-vêtement discret comportant un premier panneau, un deuxième panneau fixé au premier panneau au niveau de coutures latérales, un assemblage absorbant fixé entre le premier panneau et le deuxième panneau, une ouverture de ceinture et une paire d'ouvertures de jambes ;
b. un dispositif de séparation de panneau ;
c. un dispositif de support de transport ;
d. un poste de découpe de panneau pour découper le premier panneau pour définir une première partie du panneau avant et une deuxième partie du panneau avant ; et
e. un poste de liaison d'assemblage de fixation pour relier la première partie du panneau avant et de la deuxième partie du panneau avant.
